# EUROPEAN PATENT APPLICATION

(11) **EP 3 848 048 A1**
(43) Date of publication of application: **14.07.2021**
(21) Application number: 19854137.7
(22) Date of filing: 02.09.2019
(51) Int. Cl.: A61K 39/39, A61K 9/10, A61K 31/7024, A61K 31/7028, A61K 31/722, A61K 39/00, A61K 45/00, A61K 47/12, A61K 47/24, A61K 47/36, A61P 37/04, A61P 43/00

(54) **IMMUNOSTIMULANT, METHOD FOR PRODUCING SAME, AND KIT AND VACCINE USING IMMUNOSTIMULANT**

(30) Priority: 31.08.2018 JP 2018163914
(71) Applicant: Zenoaq Resource Co., Ltd., Koriyama-shi, Fukushima 963-0196 (JP)
(72) Inventor: KODAMA, Hiroaki, Koriyama-shi, Fukushima 963-0196 (JP); KOYANAGI, Masanori, Koriyama-shi, Fukushima 963-0196 (JP); OGAWA, Jun, Koriyama-shi, Fukushima 963-0196 (JP); ISHII, hironori, Koriyama-shi, Fukushima 963-0196 (JP)
(74) Representative: Bittner, Thomas L.
(86) International application number: PCT/JP2019/034372
(87) International publication number: WO 2020/045679

(57) **Abstract**

The present invention provides, as an immunostimulator having improved immunostimulatory activity, an immunostimulator containing chitosan and a toll-like receptor (TLR) ligand.

## Description

The present invention relates to an immunostimulator and use of the immunostimulator.

### Background Art

An innate immune system is triggered by recognition by a pattern recognition receptor (PRR) of a characteristic molecular pattern (pathogen-associated molecular pattern: PAMP) that is conserved in a bacterium, virus, or the like. A typical PRR is a toll-like receptor (TLR). In a case where a microorganism is infected, a plurality of TLR ligands act as pathogen-associated molecular patterns (PAMP), and thus contribute to activation of an innate immune system. Initiation of an innate immune system leads to activation of an acquired immune system, and thus a pathogen is specifically recognized and memorized. This effectively eliminates the same pathogen which is encountered repeatedly. Glycolipids (a-galactosylceramide: α-GalCer) which are presented in association with CD1d on surfaces of antigen presenting cells activate NKT cells. The NKT cells thus activated bridge the innate immune system with the acquired immune system so as to amplify functions of both the innate immune system and the acquired immune system.

An effective case of vaccine-induced activation of an antigen presenting cell is that an adjuvant and an antigen act together on the same antigen presenting cell. In this case, the antigen and the adjuvant are effectively protected with fine particles and the like so that the antigen and the adjuvant would not be decomposed (Hafner AM, Cortheesy B, Merkle HP. Particulate formulations for the delivery of poly(I:C) as vaccine adjuvant. Adv. Drug Deliv. Rev. 65 (2013) 1386-1399). Poly(lactic-co-glycolic acid) (PLGA) nanoparticles are widely used as the fine particles for protecting the antigen and the adjuvant because such PLGA nanoparticles have biocompatibility and biodegradability ("Silva AL, Soema PC, Slutter B, Ossendorp F, Jiskoot W. PLGA particulate delivery system for subunit vaccines: Linking particle properties to immunogenicity. Hum. Vaccin. Immunother. 12 (2016) 1056-1069" and "Dolen T, Kreutz M, Gileadi U, Tel J, Vasaturo A, van Dinther EAW, van Hout-Kuijer MA, Cerundolo V, Figdor CG. Co-delivery of PLGA encapsulated invariant NKT cell agonist with antigenic protein induce strong T cell-mediated antitumor immune responses. Oncoimmunol. 5 (2016) e1068493"). However, in order to produce the PLGA nanoparticles, it is necessary to use an organic solvent, and it is also necessary to carry out a complicated operation such as emulsification.

Document WO 2018/025926 discloses an immunostimulator using particulate chitosan produced with use of an anionic surfactant.

The particulate chitosan disclosed in WO 2018/025926 can be easily prepared using an inexpensive source material, and the particulate chitosan itself has strong adjuvant activity. In addition, the particulate chitosan is presumed to protect the antigen from being degraded in a living body, and is also excellent as a means of allowing the antigen to enter an antigen presenting cell.

However, for example, in a case where the particulate chitosan is used in combination with other adjuvant or the like for the purpose of further enhancing immunostimulatory activity or changing the quality of immunostimulatory activity, the property as an adjuvant exhibited by the particulate chitosan itself may be rather disadvantageous.

### Summary

The present invention is accomplished in view of the above problems, and its object is to provide an immunostimulator which is based on chitosan and has improved and enhanced immunostimulatory activity.

For solving the object, an immunostimulator of claim 1 is provided. Further, a kit of claim 9, a vaccine of claim 10 and a method for producing an immunostimulator of claim 12 are provided.

The inventors conducted extensive studies to solve the above described problems. As a result, the inventors of the present invention have found the fact below through a more detailed analysis of adjuvant activity itself possessed by the particulate chitosan and studies on a combination of the particulate chitosan and other adjuvants and the like on the basis of the analysis. That is, the inventors of the present invention have found that a combination of the particulate chitosan and a particular immunostimulatory substance provides improved and enhanced adjuvant activity. On the basis of this finding, the present invention is conceived.

In order to attain the object, the present invention encompasses, for example, the following features.
(1) An immunostimulator including: particulate chitosan; and a toll-like receptor (TLR) ligand.
(2) A kit for producing an immunostimulator, the kit including: chitosan which is contained in a container and carries an anionic surfactant; and an instruction manual for the kit, the chitosan being particulate chitosan, and the instruction manual indicating that the immunostimulator is produced by causing the particulate chitosan to carry a TLR ligand.
(3) A vaccine including the immunostimulator described in (1).
(4) A method for producing an immunostimulator, the method including a step of causing particulate chitosan to carry a TLR ligand.

According to an aspect of the present invention, it is possible to provide the immunostimulator having improved immunostimulatory activity.

### Brief Description of Drawings

- Fig. 1: shows a result of Example 1 of the present invention.
- Fig. 2: shows a result of Example 2 of the present invention.
- Fig. 3: shows a result of Example 3 of the present invention.
- Fig. 4: shows a result of Example 4 of the present invention.
- Fig. 5: shows a result of Example 5 of the present invention.
- Fig. 6: shows a result of Example 6 of the present invention.
- Fig. 7: shows a result of Referential Example of the present invention.
- Fig. 8: shows a result of Example 7 of the present invention.
- Fig. 9: shows a result of Example 8 of the present invention.
- Fig. 10: shows a result of Example 9 of the present invention.
- Fig. 11: shows a result of Example 10 of the present invention.
- Fig. 12: shows a result of Example 11 of the present invention.
- Fig. 13: shows a result of Example 12 of the present invention.

### Description of Embodiments

### 1. Immunostimulator

An immunostimulator in accordance with the present embodiment contains particulate chitosan and a toll-like receptor (TLR) ligand.

The immunostimulator in accordance with the present embodiment can contain the particulate chitosan and the TLR ligand in any form, and it is preferable that the particulate chitosan retains the TLR ligand. In a case where the immunostimulator in accordance with the present embodiment is used, for example, in the form of a vaccine and the particulate chitosan retains the TLR ligand, the TLR ligand is less likely to be degraded in a living body, as compared to a case where the TLR ligand is present alone. Similarly, a STING agonist (e.g., interferon gene stimulating factor), as well as an antigen and α-GalCer described later, and the like, can be retained by the particulate chitosan so that these substances are less likely to be degraded in a living body. In addition, various substances (e.g., TLR ligand, antigen, α-GalCer, and the like) retained by the same particulate chitosan can be delivered together to an antigen presenting cell.

The form in which the particulate chitosan retains various substances (e.g., TLR ligand, antigen, α-GalCer, STING agonist, and the like) can be, for example: (1) a form in which the substances are incorporated into gaps between molecular chains of chitosan constituting the particles; and/or (2) a form in which a bond is formed between an amino group possessed by chitosan and any of the substances (e.g., a TLR ligand such as a polynucleotide which is particularly negatively charged).

As used herein, the term "immunostimulator" refers to a molecule, a substance, or a composition each of which has the ability to specifically or non-specifically change, increase, induce, reinduce, enhance, or initiate an antigen-specific immune response. The immunostimulator in accordance with an aspect of the present invention encompasses those that are capable of enhancing either humoral immunity or cell-mediated immunity and those that are capable of enhancing both humoral immunity and cell-mediated immunity. As used herein, the term "immunostimulator" intends an agent which has at least a function as an adjuvant.

### [Particulate chitosan]

The particulate chitosan refers to chitosan in the form of particles. The particulate chitosan, for example, is a particle in which molecules of chitosan are formed in a highly aggregated manner and is in a state of being suspended in water. The particulate chitosan itself has an ability to induce higher antibody production. It is also presumed that chitosan in the particulate form allows chitosan to more easily enter an antigen presenting cell because, for example, (1) chitosan in the particulate form is easily recognized as a foreign substance in a living body and (2) chitosan in the particulate form is excellent in affinity with a cell surface because chitosan is positively charged. Note that, as described later, chitosan can be made into fine particles by using, for example, an anionic surfactant.

A weight-average molecular weight of the particulate chitosan is appropriately selected according to a use of an immunostimulator which is eventually obtained. The particulate chitosan in accordance with the present embodiment tends to have a greater immunostimulating effect as the molecular weight of particulate chitosan becomes lower. Note, however, that chitosan having a low molecular weight that is equal to or lower than a certain molecular weight, for example, chitosan having a molecular weight less than 5k, necessitates relatively complicated production processes.

In contrast, chitosan having a high molecular weight that is equal to or greater than a certain molecular weight, for example, chitosan having a molecular weight greater than 1000k, tends to have a high viscosity of solution in particle making.

As such, in order to achieve a great immunostimulating effect while offering good handleability, easiness of industrial production, easy availability, and high commercial applicability, the weight-average molecular weight of chitosan constituting particulate chitosan is preferably 5k or more and 1000k or less, more preferably 5k or more and 500k or less, most preferably 10k or more and 100k or less.

There is no particular limitation on a preferable particle diameter of the particulate chitosan. In view of entrance into a cell, a smaller particle diameter may be preferable. For example, the particle diameter of the particulate chitosan is 500 nm or less, or alternatively, the particle diameter can be 300 nm or less. A lower limit of the particle diameter of the particulate chitosan is not particularly limited. The lower limit can be, for example, 10 nm or more, 100 nm or more, 150 nm or more, or 200 nm or more. From the viewpoint of ease of production, the particle diameter of the particulate chitosan may be preferably 100 nm or more. The particle diameter of the particulate chitosan can be measured by, for example, a laser diffraction scattering method.

A concentration of the particulate chitosan contained in the immunostimulator in accordance with the present embodiment can be appropriately determined according to solubility or viscosity of chitosan, a type of a subject to which the immunostimulator is to be administered, and the like. In a case where the immunostimulator is in the form of a liquid, the concentration of the particulate chitosan is preferably, for example, 0.1 mg/ml or more and 50 mg/ml or less. From the viewpoint of reduction in volume (liquid amount) of the immunostimulator, a higher concentration of the particulate chitosan in the immunostimulator is preferable. In accordance with solubility, viscosity, or the like of chitosan, an upper limit of the concentration of the particulate chitosan can be, for example, 50 mg/ml or less, 45 mg/ml or less, 40 mg/ml or less, 35 mg/ml or less, 30 mg/ml or less, 25 mg/ml or less, 20 mg/ml or less, 15 mg/ml or less, or 10 mg/ml or less. For example, the immunostimulator contains the particulate chitosan at a concentration which is 80% or more of the upper limit concentration indicated above.

A method for producing the particulate chitosan will be described in the section "2. Method for producing immunostimulator".

The chitosan forming the particulate chitosan can be a chitosan derivative which has been derivatized. That is, the particulate chitosan is formed by chitosan and/or the chitosan derivative. The chitosan derivative is composed of a chitosan main chain and a side chain component. The side chain component is formed by a saccharide having a reducing end, an amino acid (derivative), and/or other compounds.

Examples of the saccharide include those derived from aldoses and those derived from ketoses, in each of which one or more constituent saccharide units are contained. More specific examples of the saccharide include monosaccharides such as: pentoses and hexoses, e.g., glucose, fucose, mannose, arabinose, galactose, xylose, erythrose, heptulose, hexulose, pentulose, and the like; amino sugars such as glucosamine, N-acetyl glucosamine (e.g., N-acetyl-D-glucosamine), and galactosamine; and saccharide derivatives such as uronic acids and deoxysugars. Specific examples of the saccharide further include disaccharides and polysaccharides each composed of a saccharide chain which is a combination of any of the above-listed monosaccharides as constituents, such as: maltose, isomaltose, lactose, melibiose, and maltotriose; and various oligosaccharides. Specific examples of the saccharide further include: natural polysaccharides such as pullulan, inulin, amylose, amylopectin, dextran, dextrin, and starch; and degraded or isomerized versions thereof and derivatives thereof. One or more of these saccharides in combination can be used in the production. These saccharides as source materials may be in the form of a hydrate.

Examples of the amino acid (derivative) include lysine (e.g., L-lysine), carnitine, arginine, proline, and histidine. Among these, lysine and carnitine are suitable as the amino acid (derivative).

The above other compounds can be, for example, polyethylene glycol. One exemplary polyethylene glycol is methoxy PEG-aldehyde.

As an example, the chitosan derivative may preferably be structured such that: a saccharide with a free carboxyl end group is bound, via a Schiff base by reductive alkylation, to the amino group bound to carbon at 2-position of a glucosamine unit constituting a saccharide unit of chitosan; or an amino acid with a free carboxyl end group forms an amide bond with the amino group bound to carbon at 2-position of a glucosamine unit constituting a saccharide unit of chitosan through a condensation reaction.

In the chitosan main chain, the number of constituent saccharide units is, for example, 30 to 12000, or can be 60 to 6000, or can be 120 to 3000. The degree of substitution, which indicates the extent to which the amino groups at 2-positions of the saccharide units of the chitosan main chain are substituted by side chains, can be appropriately selected according to the type of chitosan derivative and the final use of the chitosan derivative. The degree of substitution can be, for example, 0.005 to 0.5, or can be 0.005 to 0.3. The degree of substitution may be determined based on a known technique (e.g., sugar composition analysis), and may be calculated, for example, by NMR spectrometry. Details (i.e., types and production method) of the chitosan derivative are disclosed in, for example, WO2018/025926 (incorporated herein by reference).

As is also shown in Examples, the immunostimulator containing the particulate chitosan formed by the chitosan derivative has an effect of shrinking tumors and an effect of increasing a blood antibody titer, as with the immunostimulator containing the particulate chitosan formed by chitosan.

### [TLR ligand]

A toll-like receptor (TLR) is a family of protein that senses a microbial product and initiates an acquired immune response. A TLR ligand is a substance which binds to TLR and activates the TLR. In a typical example, activation of TLRs involves dimerization of TLRs.

Ten TLRs have been identified in a human, and 13 TLRs have been identified in a mouse. Among the TLRs identified in a human, TLR1, TLR2, TLR4, TLR5, and TLR6 are expressed on surfaces of cells. Meanwhile, TLR3, TLR7/8, and TLR9 are expressed in an endoplasmic reticulum.

The immunostimulator in accordance with the present embodiment preferably contains a human TLR ligand. Examples of the human TLR ligand which is known in this technical field and is useful in the immunostimulator in accordance with the present embodiment include, but not limited to: proteinaceous ligands such as glycoprotein, lipoprotein, glycopeptide, and lipopolypeptide; polysaccharide ligands such as lipopolysaccharide; nucleic acid ligands such as DNA and RNA; and low-molecular ligands such as an imidazoquinoline compound; and the like. More specifically, examples of the human TLR ligand include: lipoprotein and lipopolysaccharide (TLR2 ligand or TLR4 ligand); double-stranded DNA (TLR3 ligand); flagellin which is a proteinaceous ligand (TLR5 ligand); imiquimod and resiquimod (R848) which are imidazoquinoline compounds; single-stranded RNA (TLR7 ligand or TLR8 ligand); a CpG sequence (TLR9 ligand) which is a nucleic acid ligand; and the like.

Further specifically, examples of the human TLR ligand include:
Pam3Cys (TLR1/2 ligand, proteinaceous ligand);
CFA (TLR2 ligand, nucleic acid ligand);
MALP2 (TLR2 ligand, proteinaceous ligand);
Pam2Cys (TLR2 ligand, proteinaceous ligand);
FSL-1 (TLR2 ligand, proteinaceous ligand);
Hib-OMPC (TLR2 ligand, proteinaceous ligand);
polyriboinosinic-polyribocytidylic acid (poly(I:C)) (TLR3 ligand, nucleic acid ligand);
polyadenosine-polyuridylic acid (PolyAU) (TLR3 ligand, nucleic acid ligand);
polyinosine-polycytidylic acid stabilized with poly-L-lysine and carboxymethyl cellulose (Hiltonol (registered trademark)) (TLR3 ligand, nucleic acid ligand);
monophosphoryl lipid A (MPL) (TLR4 ligand);
bacterial flagellin (TLR5 ligand, proteinaceous ligand);
R848 (TLR7/8 ligand);
loxoribine (TLR7/8 ligand); and
unmethylated CpG dinucleotide (hereinafter referred to as "CpG" and can also be commonly referred to as "CpG-ODN") (TLR9 ligand, nucleic acid ligand).

Among these, the TLR3 ligand, the TLR4 ligand, the TLR7/8 ligand and the TLR9 ligand are preferable. Among these, poly(I:C), MPL, R848 and CpG are preferable, and a combination of two or more of these may be more preferable. A preferred combination can be, for example, a combination of MPL and CpG, a combination of poly(I:C) and CpG, and a combination of MPL and poly(I:C). A combination of MPL, poly(I:C) and CpG may be more preferable. As is also shown in Examples, according to a particular embodiment (of the immunostimulator used to treat or prevent a cancer), the TLR ligands that are superior in tumor-shrinking effect can be CpG, R848, MPL, and poly(I:C) (in particular, CpG and R848) (see Example 11).

Note that the TLR ligand can be naturally derived or synthetically obtained.

A concentration of the TLR ligand contained in the immunostimulator in accordance with the present embodiment can be appropriately determined according to a type of a subject to which the immunostimulator is to be administered, and the like. In a case where the immunostimulator is in the form of a liquid, the concentration of the TLR ligand is preferably, for example, 3 µg/ml or more and 50 mg/ml or less. From the viewpoint of reduction in volume (liquid amount) of the immunostimulator, a higher concentration of the TLR ligand in the immunostimulator is preferable. An upper limit of the concentration of the TLR ligand can be, for example, 50 mg/ml or less, 45 mg/ml or less, 40 mg/ml or less, 35 mg/ml or less, 30 mg/ml or less, 25 mg/ml or less, 20 mg/ml or less, 15 mg/ml or less, or 10 mg/ml or less. A lower limit of the concentration of the TLR ligand can be, for example, 3 µg/ml or more, 10 µg/ml or more, 25 µg/ml or more, 50 µg/ml or more, 75 µg/ml or more, 100 µg/ml or more. For example, the immunostimulator contains the TLR ligand at a concentration which is 80% or more of the upper limit concentration indicated above.

### [Anionic surfactant]

The immunostimulator in accordance with the present embodiment preferably contains an anionic surfactant. The anionic surfactant contains an anionic group and a hydrophobic group. The hydrophobic group is, for example, a substituted or unsubstituted group, and can be a saturated or unsaturated C2-C22 hydrocarbon group.

The anionic surfactant is preferably complexed with chitosan. The state where "the anionic surfactant is complexed with chitosan" refers to, for example, a state in which an anionic group of the anionic surfactant and an amino group of chitosan form a bond. In a case where the anionic surfactant and chitosan are complexed, the TLR ligand, the antigen, the α-GalCer, and the like are presumed to be (1) caught in gaps between molecular chains of chitosan or in gaps between molecular chains of the chitosan-anionic surfactant or (2) retained by a bond formed with a cationic (e.g., derived from the amino group of chitosan), anionic, and hydrophobic complex of the anionic surfactant and chitosan. That is, it is presumed that the substances can be retained by the particulate chitosan more firmly by utilizing hydrophobic bonds in addition to ionic bonds. The immunostimulator in accordance with the present embodiment can contain wide varieties of TLR ligands, antigens, α-GalCer, and the like.

The anionic surfactant in accordance with the present embodiment is preferably at least one selected from the group consisting of phospholipids, C10-C22 fatty acids, and salts of the C10-C22 fatty acids. The anionic surfactant can be a single kind of anionic surfactant or can be a combination of two or more different kinds of anionic surfactant (i.e., both of a phospholipid and a C10-C22 fatty acid or a salt of the C10-C22 fatty acid).

The form of the anionic surfactant in the immunostimulator in accordance with the present embodiment is not particularly limited, and can be, for example, micellar in a liquid or can be solid.

### (Phospholipid)

Examples of the phospholipids include phosphoglycerides, sphingolipids, cephalin, and mixtures thereof, totally synthetic phospholipids, lecithin, and lysolecithin. Lecithin in the field of chemistry is equivalent to phosphatidylcholine, and thus refers to phosphatidylcholine in this specification. A phospholipid can preferably be lecithin or lysolecithin.

More specifically, examples of the lecithin include soybean lecithin, egg yolk lecithin, hydrogenated soybean lecithin (HSPC), and hydrogenated egg yolk lecithin (HEPC). Examples of the phosphoglycerides include phosphatidylcholine, phosphatidylethanolamine, phosphatidylserine, phosphatidylglycerin, phosphatidylinositol, lysophosphatidylcholine, and phosphatidic acid. Examples of the sphingolipids include sphingomyelin. Note that phospholipids that are not soluble enough in water can be dissolved in an organic solvent such as ethanol and then subjected to the production of an immunostimulator.

### (Fatty acid and salt thereof)

Fatty acids and salts thereof include C10-C22 fatty acids, salts thereof, and glycerides thereof. A fatty acid may be a saturated fatty acid or an unsaturated fatty acid. The fatty acid or a salt thereof is preferably a C10-C22 fatty acid or a salt thereof, more preferably a C12-C18 fatty acid or a salt thereof.

Examples of the fatty acids include myristic acid, palmitic acid, stearic acid, arachidic acid, behenic acid, lignoceric acid, myristoleic acid, palmitoleic acid, oleic acid, vaccenic acid, icosenoic acid, docosenoic acid, tetracosenoic acid, linoleic acid, α-linolenic acid, icosadienoic acid, icosatetraenoic acid, icosatrienoic acid, icosapentaenoic acid, docosahexaenoic acid, capric acid, lauric acid, γ-linolenic acid, and arachidonic acid.

Examples of salts of the above-listed fatty acids include sodium salts and potassium salts. Note that fatty acids that are not soluble enough in water can be dissolved in an organic solvent such as ethanol and then subjected to the production of an immunostimulator.

For example, the immunostimulator in accordance with the present embodiment contains sodium oleate or sodium laurate as an anionic surfactant.

A preferred amount of the anionic surfactant contained in the immunostimulator in accordance with the present embodiment will be described later. The amount of the anionic surfactant contained in the immunostimulator is determined by, for example, determining a final concentration of the anionic surfactant in a chitosan solution prepared in a process of producing an immunostimulator.

### [Antigen]

The immunostimulator in accordance with the present embodiment can contain an antigen.

The antigen is not particularly limited and can be, for example, a cell, a bacterium, a virus, a nucleic acid, a protein, a carbohydrate, a lipid, a glycoprotein, a lipoprotein, a glycolipid, an oligopeptide, a polypeptide, a hormone, metal ions, and the like. Note that an antigen that also functions as a TLR ligand is regarded as a TLR ligand in this specification. More specifically, examples of the antigen include: cancer cells, cells in which specific genes are forcibly expressed; bacteria (such as 0157, salmonella, tubercle bacillus), viruses (such as influenza virus, measles virus, polio virus) and nucleic acids which encode antigenic proteins; tumor markers such as albumin, α-fetoprotein (AFP), carcinoembryonic antigen (CEA), CA19-9, basic fetal protein (BFP), pancreatic oncofetal antigen (POA), aldolase, alkaline phosphatase, γ glutamyl transpeptidase, neuron-specific enolase, 5'-nucleotide phosphodiesterase isozyme V (5'-NPD-V), and abnormal prothrombin (PIVKA-II); immunoglobulins such as IgM, IgG, IgA, IgE, and IgD; hepatitis B virus-associated antigen, hepatitis C virus-associated antigen, and viral antigens such as influenza virus antigen; hormones such as thyroid hormone and steroid hormone; and the like. Among these, the viral antigen, the bacterial antigen, a cancer antigen, or a cancer cell lysate is preferable, and a cancer cell antigen or a cancer cell lysate is more preferable. The term "cancer antigen" in this specification refers to a gene product (e.g., a protein, a nucleic acid, and a modified adduct thereof) that is specifically highly expressed in a tumor, or to a fragment of the gene product.

An amount of the antigen that can be contained in the immunostimulator in accordance with the present embodiment can be appropriately determined according to a type of the antigen and a subject to which the immunostimulator is to be administered. For example, in a case where a known antigenic protein is used, the amount can be determined by referring to a previous report on a dosage amount of the antigen with respect to a subject to which the immunostimulator is to be administered. For example, for mammals including a cattle, a pig, a human, and the like, the amount can be, for example, 0.3 µg or more and 300 mg or less, or can be 10 µg or more and 300 µg or less.

In a case where the immunostimulator in accordance with the present embodiment is used in the form containing the antigen, it is advantageous that the TLR ligand and the antigen act together on the same antigen presenting cell. In a case where both the TLR ligand and the antigen in accordance with the present embodiment are retained on the particulate chitosan, the TLR ligand and the antigen are protected, and the TLR ligand and the antigen are difficult to separate.

### [α-GalCer]

The immunostimulator in accordance with the present embodiment can contain an α-galactoceramide (α-GalCer). The immunostimulator which contains α-GalCer acts more effectively as an immunostimulator because the α-GalCer promotes IFN-γ production.

### [Content ratios of components with respect to particulate chitosan]

In a case where the immunostimulator is in the form of a liquid, a concentration of the particulate chitosan in the immunostimulator in accordance with the present embodiment is preferably, for example, 0.1 mg/ml or more and 50 mg/ml or less. From the viewpoint of reduction in volume (liquid amount) of the immunostimulator, a higher concentration of the particulate chitosan in the immunostimulator is preferable. In accordance with solubility, viscosity, or the like of chitosan, an upper limit of the concentration of the particulate chitosan can be, for example, 50 mg/ml or less, 45 mg/ml or less, 40 mg/ml or less, 35 mg/ml or less, 30 mg/ml or less, 25 mg/ml or less, 20 mg/ml or less, 15 mg/ml or less, or 10 mg/ml or less. For example, the immunostimulator contains the particulate chitosan at a concentration which is 80% or more of the upper limit concentration indicated above.

A mass ratio of the TLR ligand to the particulate chitosan (ligand/chitosan) contained in the immunostimulator in accordance with the present embodiment varies according to an administration form. For example, in a case of CpG, the mass ratio is preferably 0.03 or more and 30 or less, more preferably 0.1 or more and 10 or less, further preferably 0.3 or more and 3 or less.

In a case where the immunostimulator in accordance with the present embodiment contains the anionic surfactant, a mass ratio of the anionic surfactant to the particulate chitosan (surfactant/chitosan) is preferably 0.05 or more and 1 or less, more preferably 0.1 or more and 0.5 or less, further preferably 0.15 or more and 0.3 or less.

In a case where the immunostimulator in accordance with the present embodiment contains the antigen, a mass ratio of the antigen to the particulate chitosan (antigen/chitosan) contained in the immunostimulator is preferably 0.01 or more and 10 or less, more preferably 0.03 or more and 5 or less, further preferably 0.1 or more and 1 or less.

In a case where the immunostimulator in accordance with the present embodiment contains α-GalCer, a mass ratio of α-GalCer to the particulate chitosan (α-GalCer/chitosan) contained in the immunostimulator is preferably 0.001 or more and 0.5 or less, more preferably 0.003 or more and 0.3 or less, further preferably 0.01 or more and 0.1 or less.

### [Other additive]

The immunostimulator in accordance with the present embodiment can further contain additives such as an excipient and various adjuncts, provided that the additive does not impair with the effect of the immunostimulator. Examples of the additive that can be contained include: trehalose, glucose, sucrose, lactose, glucose, mannitol, dextran, xylitol, maltose, fructose, glycine, citric acid, and sodium chloride; and nonionic surfactants. Such additives make it possible to prevent the instability of properties such as agglomeration or a decrease in immunostimulatory activity that would result from freeze drying or long-term preservation at low temperature.

Other examples of the additive include vitamin C, vitamin E, and the like. Such vitamins make it possible to suppress the oxidative decomposition of the fatty acid.

Examples of the adjuncts include a wetting agent, an emulsifying agent, a pH adjustor, and other adjuvants.

The pH adjustor can either be an alkaline material or an acidic material, according to need. The alkaline material for use in adjusting pH is, for example, sodium hydroxide or the like. The acidic material for use in adjusting pH is, for example, acetic acid, hydrochloric acid, or the like.

Such other adjuvants can be an adjuvant which is not contained in the immunostimulator in accordance with the present embodiment and is selected from, for example, an alum adjuvant, a Freund's complete adjuvant, a Freund's incomplete adjuvant, an oil adjuvant, saponin, cell wall skeleton constituents, lipopolysaccharide, endotoxin, ablysin, liposome, bacterial DNA, synthetic oligonucleotide, vitamin E, glycolipid, and squalene. In some cases, a combined use of two or more adjuvants provides a synergistic effect in, for example, accelerating immune response, as compared to cases where each adjuvant is used alone.

### [Other component]

In the immunostimulator in accordance with the present embodiment, a medicament can be retained by a surface of the particulate chitosan. Moreover or alternatively, the surfaces of the particulate chitosan can be coated with an anionic polymer such as a polycarboxylic acid. Examples of the polycarboxylic acid include pectin, alginic acid, polyacrylic acid, hyaluronic acid, chondroitin sulfate, and poly-γ-glutamic acid. The particulate chitosan coated with such a substance is provided with high stability to digestive enzymes.

### [Dosage form, administration form, etc. of immunostimulator]

In a case where the immunostimulator in accordance with the present embodiment is used, there is no particular limitation on dosage form of the immunostimulator, and the dosage form can be liquid, solid, semisolid, or semiliquid, preferably liquid. The immunostimulator in such a dosage form can be produced easily on the basis of any method known to those skilled in the art while employing a suitable pharmaceutical carrier according to need. In cases where the dosage form is solid, the dosage form is, for example, powder, granules, tablet, capsule, or the like. The case where the dosage form is solid also includes, for example, a form in which particulate chitosan retaining at least a TLR ligand is employed as particles. In cases where the dosage form is semisolid or semiliquid, the dosage form is, for example, ointment, lotion, cream, gel, or the like. In cases where the dosage form is liquid, examples of a solvent used include water and a buffer. Examples of the buffer include physiological saline, a phosphate buffer, and a Ringer solution. The solvent can be a mixture of two or more of the above listed solvents.

Examples of the route of administration of the immunostimulator in accordance with the present embodiment include, but are not limited to, oral, topical, subcutaneous, intramuscular, intravenous, intradermal, intraabdominal, transmucosal, and transdermal administrations. The immunostimulator is administered on the basis of a known method, for example, administered by directly injecting subcutaneously, intradermally, intravenously, intramuscularly, intraperitoneally, or the like; by spraying to an intranasal, intraoral, intrapulmonary, intravaginal, or intrarectal mucous membrane or the like; or orally administered.

### [Administration target of immunostimulator]

Examples of a subject to which the immunostimulator is to be administered include all kinds of animals. The subject is preferably a vertebrate such as a mammal or a bird, more preferably a mammal. The mammal subject is preferably a human, but can be a domestic animal, a laboratory animal, or a pet animal in some cases. Specific examples of a subject include: domestic animals such as chickens, pigs, horses, goats, sheep, and cattle; pet animals such as cats, dogs, hamsters, rabbits, and guinea pigs; mice; rats; monkeys; fish; and birds.

The immunostimulator in accordance with the present embodiment is generally administered to a subject such as an animal including a human in an amount and the number of times that are sufficient to enhance an immune system. An upper limit of a single dose (in an amount of the particulate chitosan contained in the immunostimulator) to an animal can be, for example, 50 mg or less, 45 mg or less, 40 mg or less, 35 mg or less, 30 mg or less, 25 mg or less, 20 mg or less, 15 mg or less, 10 mg or less, 1 mg or less, or a range of 0.5 mg or less (note that a mass ratio of the TLR ligand to the particulate chitosan (ligand/chitosan) varies according to the administration form and, for example, in the case of CpG, the mass ratio is 0.03 or more and 30 or less, and more specifically, examples of the mass ratio include 0.1 or more and 10 or less, 0.3 or more and 3 or less, and the like). A lower limit of the single dose (in an amount of the particulate chitosan contained in the immunostimulator) to an animal can be, for example, 3 µg or more, 30 µg or more, 300 µg or more, 3 mg or more, or 30 mg or more. The administration in such a dosage amount makes it possible to achieve a sufficient level of immunostimulatory activity while ensuring safety to the living body, without involving adverse effects that would be caused by excessive stimulation of immunity in the subject, and thus is effective.

In a case where the immunostimulator is in the form of a liquid, the concentration of the particulate chitosan is preferably, for example, 0.1 mg/ml or more and 50 mg/ml or less. From the viewpoint of reduction in volume (liquid amount) of the immunostimulator, a higher concentration of the particulate chitosan in the immunostimulator is preferable. In accordance with solubility, viscosity, or the like of chitosan, an upper limit of the concentration of the particulate chitosan can be, for example, 50 mg/ml or less, 45 mg/ml or less, 40 mg/ml or less, 35 mg/ml or less, 30 mg/ml or less, 25 mg/ml or less, 20 mg/ml or less, 15 mg/ml or less, or 10 mg/ml or less. For example, the immunostimulator contains the particulate chitosan at a concentration which is 80% or more of the upper limit concentration indicated above.

A more specific use application of the immunostimulator in accordance with the present embodiment is, for example, therapeutic or prophylactic treatment of infection with a bacterium, virus, or the like, or of an illness such as cancer, autoimmune disease, or allergy. Examples of the therapeutic or prophylactic treatment include vaccine therapy, immunotherapy for cancer or the like, desensitization therapy for allergenic substances, and the like.

### [Characteristics of immunostimulator]

The immunostimulator in accordance with the present embodiment has a remarkable immunostimulatory effect, and thus is useful in, for example, therapeutically treating a patient with a disease, illness, or disorder in which normal metabolic and immune responses weaken or are suppressed in the living body. The immunostimulator in accordance with an aspect of the present invention can be used to therapeutically or prophylactically treat a subject such as an animal or a human at high risk of developing a disease, illness, or disorder that would result from the condition that adversely affects the immune system.

The immunostimulator in accordance with the present embodiment has an effect of synergistically stimulating humoral immunity and cell-mediated immunity. In addition, the particulate chitosan and the TLR ligand exhibit different adjuvant effects and these effects do not counteract each other. This can lead to a considerable increase in the degree of freedom of antigen selection.

Furthermore, quite high immunostimulatory activity is exhibited even with small amounts of the immunostimulator and the antigen, and this results in fewer side effects. In particular, as is clear from Examples, even when the immunostimulator is administered, only a small amount of IgE, which may be responsible for allergic reaction, is produced.

It is presumed that such properties as described above become particularly remarkable in a case where the substances (such as TLR ligand, antigen, and α-GalCer) are retained by the particulate chitosan.

### 2. Method for producing immunostimulator

The immunostimulator in accordance with the present embodiment can be produced by a method including a step of causing the particulate chitosan to retain a TLR ligand.

### [Step of preparing particulate chitosan]

### (Chitosan as source material for producing particulate chitosan)

Usually, chitosan can be prepared by deacetylating chitin (poly-β-1,4-N-acetyl glucosamine) derived from an exoskeleton of crab or shrimp by treating it with an alkali, enzyme, or the like. In this specification, chitin at least partially deacetylated is referred to as chitosan. Note that chitosan in accordance with an aspect of the present invention is not limited to naturally-occurring ones and can be a chemically synthesized one.

Chitosan used as a source material for the particulate chitosan in accordance with the present embodiment is such that a percentage of N-deacetylated units of the glucosamine units constituting the chitin molecule, that is, a degree of deacetylation, is preferably 60% or more and 100% or less, more preferably 70% or more and 100% or less. Note that the degree of deacetylation can be determined on the basis of a known technique (e.g., colloid titration method). One example of the known technique is NMR.

The weight-average molecular weight of chitosan as a source material for the particulate chitosan can be preferably approximately 5k (i.e., 5,000) or more and approximately 1000k (i.e., 1,000,000) or less, assuming that the molecular weight of each glucosamine residue of chitosan is 161, that the molecular weight of each N-acetyl glucosamine residue is 203, and that the degree of deacetylation is 60% or more and 100% or less.

The form of chitosan in the present embodiment is not particularly limited, and can be, for example, chitosan molecules in the form of particles, powder, fibers, a film, a sheet, a hydrogel, or a solution.

### (Preparation of chitosan solution)

In a case where chitosan which is not in a solution state is used as a source material, a chitosan solution is prepared by dissolving chitosan in a solvent. The solvent can be any solvent as long as chitosan can be dissolved. Examples of the solvent include acetate buffers, and aqueous organic acid solutions such as aqueous acetic acid solutions, aqueous formic acid solutions, aqueous propionic acid solutions, aqueous malic acid solutions, aqueous succinic acid solutions, and aqueous lactic acid solutions. A buffer is, for example, an acetate buffer at a final concentration of 5 mM to 50 mM (pH5.0, in physiological saline). A concentration of chitosan in the chitosan solution is not particularly limited and is, for example, preferably 0.1 mg/ml or more and 50 mg/ml or less, preferably 0.1 mg/ml or more and 30 mg/ml or less, more preferably 0.2 mg/ml or more and 20 mg/ml or less. From the viewpoint of reduction in volume (liquid amount) of the immunostimulator, it is preferable that the concentration of chitosan is high.

### (Preparation of particulate chitosan from chitosan solution)

Examples of a method for preparing the particulate chitosan include a method in which a W/O emulsion including droplets of the chitosan solution is prepared and particulate chitosan is precipitated by concentrating the droplets. A particularly preferable method is obtaining particles of chitosan with use of an anionic surfactant. The method using the anionic surfactant is simple, and it is possible to obtain particulate chitosan in which the anionic surfactant is complexed with chitosan (i.e., particulate chitosan particularly suitable for the immunostimulator; "micronized chitosan" in Examples). Hereinafter, a method using the anionic surfactant will be described more specifically.

In the method employing the anionic surfactant, it seems that particles are formed through: (1) complexing of chitosan and the anionic surfactant via an electrostatic interaction between a protonated amino group (-NH₃⁺) of chitosan and a deprotonated carboxylate ion (-COO⁻) formed through deprotonation of a carboxyl group of the anionic surfactant; and (2) association of the anionic surfactant resulting from a hydrophobic interaction (reference document: Takashi Kuroiwa, Urakami-zaidan study report, Vol. 22, pp. 16-20 (2015)).

The chitosan solution preferably has pH of neutral to slightly acidic where chitosan becomes cationic.

The preferable example of the anionic surfactant to be mixed is as described in the section of [Anionic surfactant] in "1. Immunostimulator" above.

The chitosan and the anionic surfactant can be mixed into the solvent in any order. For example, any one of the following processes can be carried out: (1) after a chitosan solution is prepared by dissolving chitosan in a solvent, an anionic surfactant is added to the chitosan solution; (2) chitosan and an anionic surfactant are added substantially simultaneously to a solvent, and preparation of a chitosan solution and preparation of particulate chitosan are carried out simultaneously; and (3) chitosan is added after an anionic surfactant is added to a solvent, and preparation of a chitosan solution and preparation of particulate chitosan are carried out simultaneously. Among these, the process of (1) is particularly preferable.

The mass ratio of chitosan to the anionic surfactant, per particle of the particulate chitosan, varies depending on the conditions such as the molecular weight of chitosan or the type of surfactant used. In one embodiment, the molar ratio of chitosan to the anionic groups of the anionic surfactant, per particle of the particulate chitosan, is preferably 1:0.05 or more and 1:1 or less, more preferably 1:0.1 or more and 1:0.5 or less, even more preferably 1:0.15 or more and 1:0.3 or less.

### [Step of causing particulate chitosan to retain TLR ligand, etc.]

A method of causing the particulate chitosan prepared as described above to retain a TLR ligand is not particularly limited. For example, (1) a TLR ligand can be brought into contact with particulate chitosan which has been prepared or (2) a TLR ligand can be brought into contact with particulate chitosan which is being prepared. In the case of (1), for example, a TLR ligand can be added to a solvent containing particulate chitosan which has been prepared. In the case of (2), for example, particulate chitosan is prepared from a chitosan solution containing a TLR ligand, and thus preparation of the particulate chitosan and a process of causing the particulate chitosan to retain the TLR ligand can be carried out simultaneously. In both cases of (1) and (2), it is preferable to use the particulate chitosan prepared by the method using the anionic surfactant. In the case of (1), the prepared particulate chitosan can be kept frozen or refrigerated.

With use of a method similar to the method of causing the particulate chitosan to retain the TLR ligand, the above-described antigen, α-GalCer, and other components can be retained by the particulate chitosan.

### [Other step]

The particulate chitosan retaining the TLR ligand and the like can be utilized as an immunostimulator together with a solvent thereof. Alternatively, it is possible that the particulate chitosan retaining the TLR ligand and the like is collected and subjected to processes such as washing, and is then used as an immunostimulator in combination with a carrier or the like as needed.

### 3. Kit

An aspect of the present invention also provides a kit for producing an immunostimulator. The kit includes: (1) particulate chitosan which is contained in a container and retains an anionic surfactant; and (2) an instruction manual for the kit. The container can be, for example, a sealable bag, a sealed container with a lid, or the like. The particulate chitosan in the container can be in a state of being dispersed in a liquid. The instruction manual can be, for example, printed on a recording medium such as paper or on the container, or can be electronically stored in an information-recording medium or on the Internet.

The instruction manual indicates that the immunostimulator is produced by causing the particulate chitosan to retain a TLR ligand. More specifically, for example, the instruction manual indicates a method of causing particulate chitosan to retain a TLR ligand as described in the section [Step of causing particulate chitosan to retain TLR ligand, etc.] in "2. Method for producing immunostimulator" above.

Note that the "anionic surfactant", the "particulate chitosan", and the "TLR ligand" are the same as those described in "1. Immunostimulator" above. It is preferable that the particulate chitosan retaining the anionic surfactant is one in which the anionic surfactant and chitosan are complexed.

The kit can further include, if necessary, any of various TLR ligands; antigens; instruments (e.g., syringe) for intracavitary administration or blood sampling; reagents and instruments (e.g., pipette, 96-well microplate) used in measuring an antibody titer; and the like. The kit can be used in production of the immunostimulator, and thus in production of a vaccine and the like for stimulating immunity.

### 4. Use of immunostimulator

### [Vaccine]

The immunostimulator in accordance with the present embodiment can also be used as a vaccine. The vaccine in this specification refers to a pharmaceutical composition which contains a pathogen itself or a specific antigen derived from the pathogen and which is intended to prophylactically or therapeutically treat a specific disease, illness, or disorder. The pharmaceutical composition is a concept including not only a pharmaceutical composition for a human but also a pharmaceutical composition for an animal. A type of antigen contained in the vaccine is selected according to a particular disease or the like to be prevented or treated. Examples of the antigen include those described in the section [Antigen] in "1. Immunostimulator" (e.g., the cancer antigen and the cancer cell lysate). Note that the vaccine can be a vaccine composition which is a mixture of various kinds of vaccines and which targets a plurality of specific diseases and the like.

In accordance with the foregoing paragraphs, in a case where the cancer antigen or the cancer cell lysate is selected as examples of the antigen, the specific disease, illness, or disorder is cancer, and the vaccine can be utilized for prevention or treatment of cancer (i.e., the vaccine is a cancer vaccine (see Examples)).

With the vaccine in accordance with the present embodiment, as compared with conventional adjuvants, it is possible to achieve a much higher level of immunostimulatory activity and a higher neutralizing antibody titer, even with the use of smaller amounts of immunostimulator and antigen. Since the adjuvant and the antigen are contained in smaller amounts, the resulting vaccine does not inflict excessive stimulations to the living body, is highly safe, and is also less costly.

The particulate chitosan contained in the immunostimulator is biodegradable and is low-toxic to the living body. Therefore, the particulate chitosan is completely decomposed and disappears over a moderate period of time. As such, the particulate chitosan may not remain within the living body to adversely affect the health of the living body, and thus can be used safely.

A Freund's adjuvant, which has conventionally been widely used for research, sometimes causes an inflammation and death of tissue at the administration site of a host animal. In contrast, the immunostimulator in accordance with the present embodiment can be administered without inflicting a severe pain on animals, and can be produced safely, less costly, and easily. Thus, the immunostimulator in accordance with the present embodiment offers a very high level of commercial value.

### [Food additive]

The immunostimulator in accordance with the present embodiment can also be used as an additive for a food or beverage. In a case where the immunostimulator in accordance with the present embodiment is used as an additive for a food or beverage, it is possible to provide a food or beverage composition having immunostimulatory activity. Examples of the food or beverage composition having immunostimulatory activity include a food or beverage for one or more specified health uses, a food or beverage with one or more functional claims, or a food or beverage with one or more nutrient function claims, each of which bears a claim of immunostimulation, immune activation, immunity enhancement, or the like.

### 5. Aspects of the present invention can also be expressed as follows.

As is clear from the above descriptions, the present invention encompasses the following features:
<1> An immunostimulator including: particulate chitosan; and a toll-like receptor (TLR) ligand.
<2> The immunostimulator described in <1>, further including an anionic surfactant.
<3> The immunostimulator described in <2>, in which the anionic surfactant is at least one selected from the group consisting of phospholipids, C10-C22 fatty acids, and salts of the C10-C22 fatty acids.
<4> The immunostimulator described in <3>, in which: the phospholipids are lecithin or lysolecithin; and the C10-C22 fatty acids and the salts of the C10-C22 fatty acids are sodium oleate or sodium laurate.
<5> The immunostimulator described in any one of <1> through <4>, in which the particulate chitosan has a weight-average molecular weight of 5k or more and 1000k or less, and has a particle diameter of 500 nm or less.
<6> The immunostimulator described in any one of <1> through <5>, in which the TLR is at least one selected from the group consisting of TLR3, TLR4, TLR7, TLR8, and TLR9 of human.
<7> The immunostimulator described in any one of <1> through <6>, further including an antigen.
<8> The immunostimulator described in any one of <1> through <7>, further including α-GalCer.
<9> A kit for producing an immunostimulator, the kit including: chitosan which is contained in a container and retains an anionic surfactant; and an instruction manual for the kit, the chitosan being particulate chitosan, and the instruction manual indicating that the immunostimulator is produced by causing the particulate chitosan to retain a TLR ligand.
<10> A vaccine containing the immunostimulator described in any one of <1> through <8>.
<11> The vaccine described in <10> which is a cancer vaccine.
<12> A method for producing an immunostimulator, the method including a step of causing particulate chitosan to retain a TLR ligand.

The following description will discuss Examples, and the embodiment of the present invention will be described in more detail. The present invention is of course not limited to Examples below, and various aspects may be employed for details. Further, the present invention is not limited to the embodiment, but can be altered by a skilled person in the art within the scope of the claims. The present invention also encompasses in its technical scope any embodiment based on an appropriate combination of the technical means disclosed. All literatures described in this specification are incorporated herein as reference literatures.

### Examples

### [Example 1]

### (Preparation of micronized chitosan dispersion solution)

Chitosan (FL-80, available from KOYO CHEMICAL CO., LTD., degree of deacetylation 90%, weight-average molecular weight 30000, Lot 0507-27) in an amount of 500 mg was dispersed in 25 ml of distilled water, and 200 µl of acetic acid was added and the chitosan was dissolved. To the chitosan solution, sodium oleate (i.e., an anionic surfactant) was added in an amount which was an equivalent weight of 0.28 with respect to an amino group of chitosan, and thus a solution in which micronized chitosan at 0.1 wt% was suspended in 5 mM acetate buffered saline (pH5.0) (hereinafter, referred to as "solution A") was obtained. Further, 1 µl of ovalbumin (OVA, available from Wako Pure Chemical Industries, Ltd.) was added as an antigen to the solution A, and thus a micronized chitosan suspension (hereinafter, referred to as "CN solution") was obtained.

### (Preparation of test solution)

To the CN solution, 10 µl of MPL (monophosphoryl lipid A (synthetic) (PHAD), available from Avanti Polar Lipids, #699800P, where PHAD is a registered trademark of Avanti Polar Lipids) was added as a TLR4 agonist, and thus a test solution M10 containing, per 100 µl, 10 µg of micronized chitosan, 1 µg of OVA and 10 µg of MPL was obtained.

Further, 1 µl of MPL was added to the CN solution, and thus a test solution M1 containing, per 100 µl, 10 µg of micronized chitosan, 1 µg of OVA and 1 µg of MPL was obtained.

### (Immunization)

Five-week-old female Balb/cAJcl mice (CLEA Japan, Inc.) were randomly selected into groups such that each group would include five mice, after one week of habituation.

Each mouse in the group 1 received 100 µl of the CN solution per administration, each mouse in the group 2 received 100 µl of the test solution M10 per administration, and each mouse in the group 3 received 100 µl of the test solution M1 per administration by intraperitoneal administrations which were carried out twice at two-week interval. One week after each immunization, blood was collected from tail veins to obtain serum samples.

### (Determination of antibody titer)

OVA was prepared in an amount of 2 µg/ml with use of 50 mM of a carbonate buffer (pH9.6). This OVA solution was dispensed into a 96-well plate in 100 µl per well to prepare an antigen solid-phase plate, and blocking was carried out with use of Block Ace (available from Snow Brand Milk Products Co., Ltd., Cat. No.: UK-B80).

Each of the serum samples which were 6000-fold diluted was added to each well in an amount of 100 µl, and was caused to react for 2 hours at a normal temperature. As a secondary antibody, an HRP-labeled anti-mouse IgG1 antibody (available from Bethyl, Cat. No.: A90-105P), an HRP-labeled anti-mouse IgG2a antibody (available from Bethyl, Cat. No.: A90-107P), or an HRP-labeled anti-mouse IgG2b antibody (available from Bethyl, Cat. No.: A90-109P) was used and caused to react for 2 hours at a room temperature.

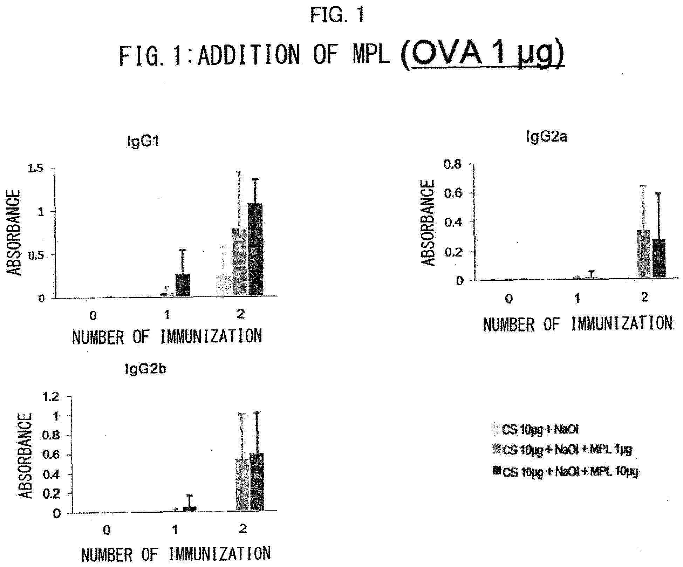

TMBZ (available from Sigma, Cat. No.: T3405) was used as a substrate and caused to react for 2 hours at a room temperature, and then the reaction was terminated with 2N sulfuric acid. Absorbance (OD value) was measured with use of a plate reader (available from BIO-RAD: model 680, Cat. No.: 168-1000) (dual: measured 450 nm, control 570 nm). The results are shown in Fig. 1.

### [Example 2]

Test solutions were obtained in a manner similar to that of Example 1, except that the amount of OVA for obtaining the CN solution of Example 1 was set to 10 µl. A test solution (100 µl) containing 10 µg of micronized chitosan, 10 µg of OVA, and 10 µg of MPL was designated "M10-10", and a test solution (100 µl) containing 10 µg of micronized chitosan, 10 µg of OVA, and 1 µg of MPL was designated "M1-10".

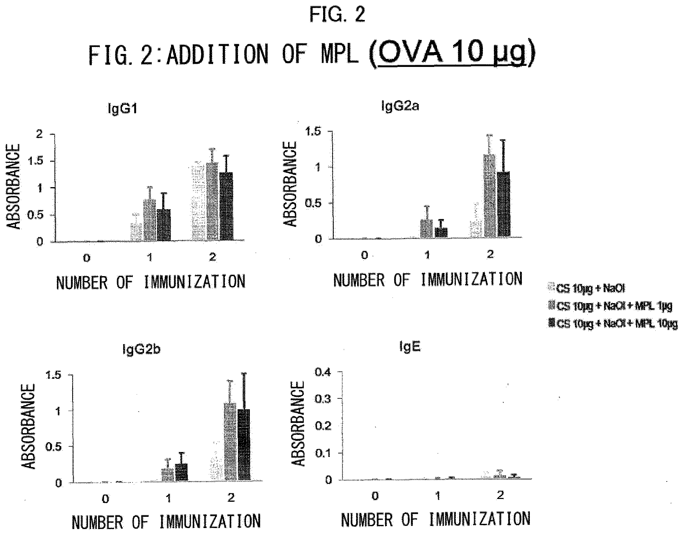

For immunizing mice, 100 µl of the test solution M10-10 was administered to each of the mice in the group 2, and 100 µl of the test solution M1-10 was administered to each of the mice in the group 3. Moreover, an operation similar to that in Example 1 was carried out, except that an HRP-labeled anti-mouse IgE antibody (available from Bethyl, Cat. No.: A90-115P) was additionally used as a secondary antibody. The results are shown in Fig. 2.

### [Example 3]

To a CN solution obtained in the same manner as that of Example 1, 30 µl of poly(I:C) (available from Invivogen, Cat. No.: tlrl-pic, hereinafter referred to as "poly(I:C)") was added instead of MPL as a TLR3 agonist, and thus a test solution P30 containing, per 100 µl, 10 µg of micronized chitosan, 1 µg of OVA and 30 µg of poly(I:C) was obtained. Moreover, a test solution P10 containing 10 µg of poly(I:C) was obtained. Furthermore, a test solution P3 containing 3 µg of poly(I:C) was obtained.

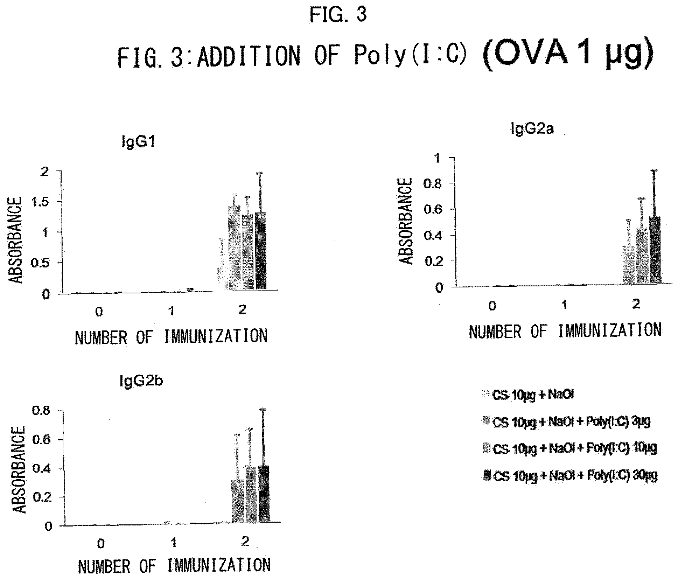

For immunizing mice, 100 µl of the test solution P30 was administered to each of the mice in the group 2, 100 µl of the test solution P10 was administered to each of the mice in the group 3, and 100 µl of the test solution P3 was administered to each of the mice in the group 4. Except for the above points, an operation similar to that in Example 1 was carried out. The results are shown in Fig. 3.

### [Example 4]

To a CN solution obtained in the same manner as that of Example 1, 15 µg of mouse-specific class B CpG (available from Invivogen, ODN 1826 VacciGrade, Cat. code: vac-1826-1) was added instead of MPL as a TLR9 agonist, and thus a test solution C15 containing, per 100 µl, 10 µg of micronized chitosan, 1 µg of OVA and 15 µg of CpG was obtained. Moreover, a test solution C5 containing 5 µg of CpG was obtained. Furthermore, a test solution C1.5 containing 1.5 µg of CpG was obtained.

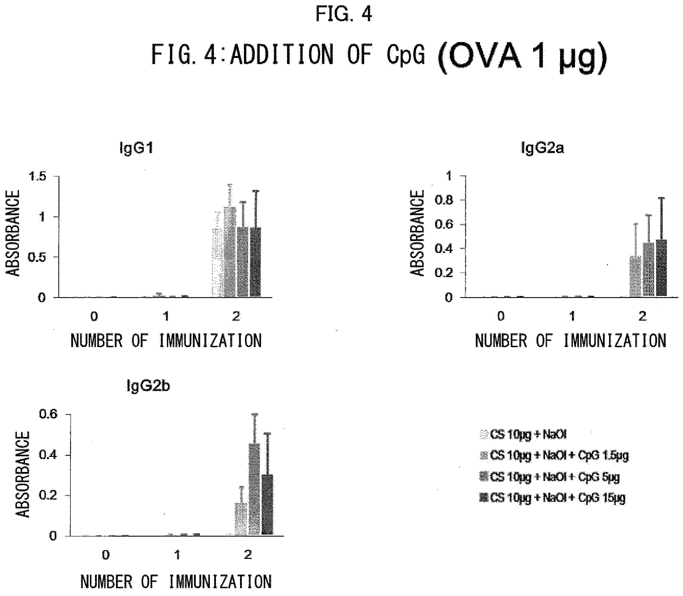

For immunizing mice, 100 µl of the test solution C15 was administered to each of the mice in the group 2, 100 µl of the test solution C5 was administered to each of the mice in the group 3, and 100 µl of the test solution C1.5 was administered to each of the mice in the group 4. Except for the above points, an operation similar to that in Example 1 was carried out. The results are shown in Fig. 4.

### [Example 5]

To a CN solution obtained in the same manner as that of Example 1, 10 µg of R848 (available from Tokyo Chemical Industry Co., Ltd., resiquimod, product code: R0197) was added instead of MPL as a TLR7/8 agonist, and thus a test solution R10 containing, per 100 µl, 10 µg of micronized chitosan, 1 µg of OVA and 10 µg of R848 was obtained. Moreover, a test solution R3 containing 3 µg of R848 was obtained. Furthermore, a test solution R1 containing 1 µg of R848 was obtained.

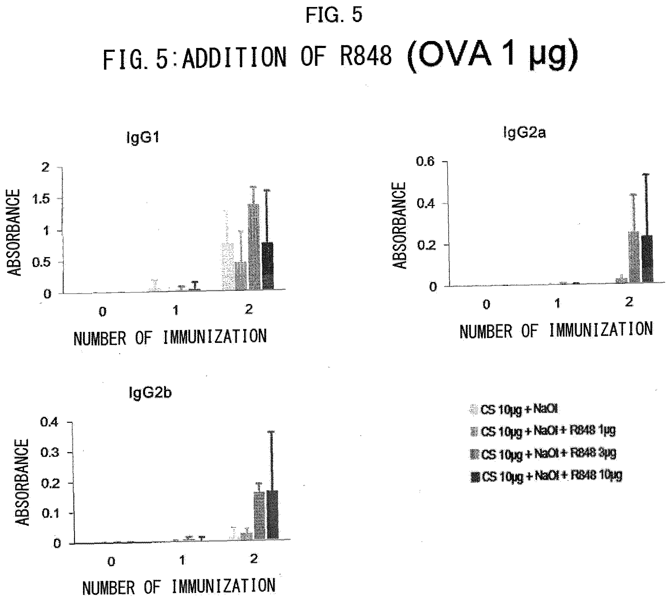

For immunizing mice, 100 µl of the test solution R10 was administered to each of the mice in the group 2, 100 µl of the test solution R3 was administered to each of the mice in the group 3, and 100 µl of the test solution R1 was administered to each of the mice in the group 4. Except for the above points, an operation similar to that in Example 1 was carried out. The results are shown in Fig. 5.

### [Example 6]

To a CN solution obtained in the same manner as that of Example 1, 3 µg of MPL and 3 µg of poly(I:C) were added, and thus a test solution MP3 containing, per 100 µl, 10 µg of micronized chitosan, 1 µg of OVA, 3 µg of MPL, and 3 µg of poly(I:C) was obtained. Moreover, a test solution MC3 was obtained which contained 3 µg MPL and 3 µg of CpG instead of 3 µg of MPL and 3 µg of poly(I:C). Moreover, a test solution MR3 was obtained which contained 3 µg MPL and 3 µg of R848 instead of 3 µg of MPL and 3 µg of poly(I:C). Moreover, a test solution PC3 was obtained which contained 3 µg poly(I:C) and 3 µg of CpG instead of 3 µg of MPL and 3 µg of poly(I:C). Moreover, a test solution PR3 was obtained which contained 3 µg poly(I:C) and 3 µg of R848 instead of 3 µg of MPL and 3 µg of poly(I:C). Moreover, a test solution CR3 was obtained which contained 3 µg CpG and 3 µg of R848 instead of 3 µg of MPL and 3 µg of poly(I:C).

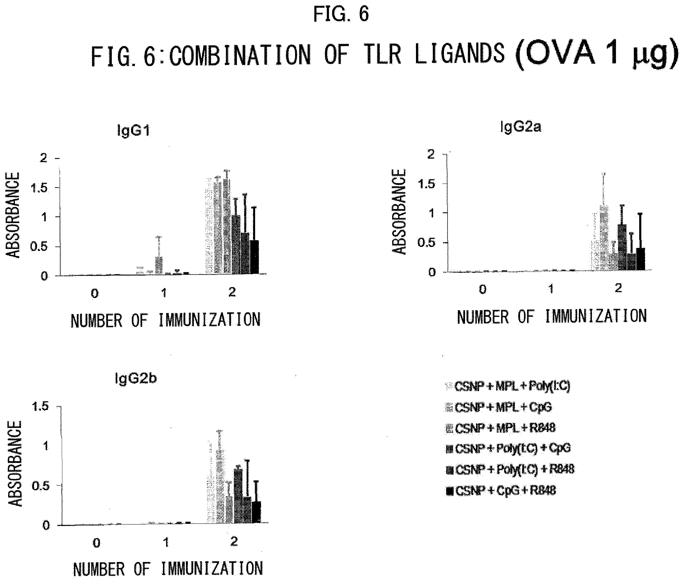

For immunizing mice, 100 µl of the test solution MP3 was administered to each of the mice in the group 1, 100 µl of the test solution MC3 was administered to each of the mice in the group 2, 100 µl of the test solution MR3 was administered to each of the mice in the group 3, 100 µl of the test solution PC3 was administered to each of the mice in the group 4, 100 µl of the test solution PR3 was administered to each of the mice in the group 5, and 100 µl of the test solution CR3 was administered to each of the mice in the group 6. Except for the above points, an operation similar to that in Example 1 was carried out. The results are shown in Fig. 6.

### [Referential Example]

To a CN solution obtained in the same manner as that of Example 1, 1 µg of α-GalCer (available from Funakoshi Co., Ltd., product code: KRN7000) was added instead of MPL as a glycolipid for activating NKT cells, and thus a test solution α1 containing, per 100 µl, 10 µg of micronized chitosan, 1 µg of OVA and 1 µg of α-GalCer was obtained. Moreover, a test solution α0.1 containing 0.1 µg of α-GalCer was obtained. Furthermore, a test solution α0.01 containing 0.01 µg of α-GalCer was obtained.

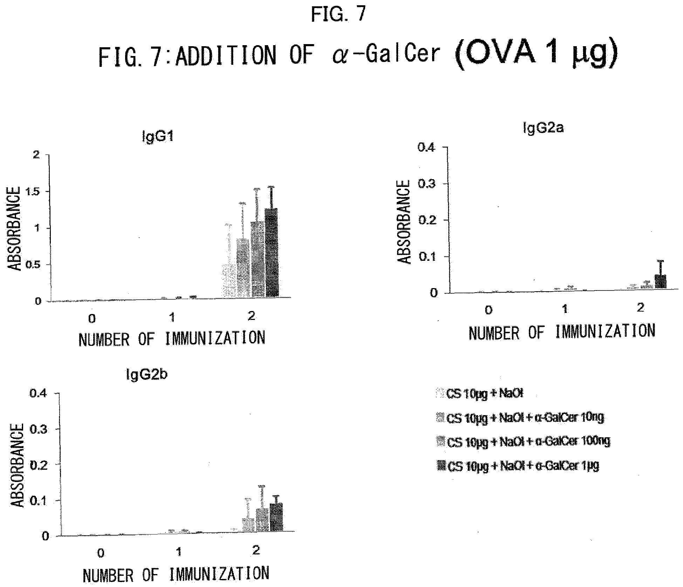

For immunizing mice, 100 µl of the test solution α1 was administered to each of the mice in the group 2, 100 µl of the test solution α0.1 was administered to each of the mice in the group 3, and 100 µl of the test solution α10.01 was administered to each of the mice in the group 4. Except for the above points, an operation similar to that in Example 1 was carried out. The results are shown in Fig. 7.

### [Example 7]

To a CN solution obtained in the same manner as that of Example 1, 3 µg of MPL, 3 µg of poly(I:C), and 3 µg of α-GalCer were added, and thus a test solution MPα3 containing, per 100 µl, 10 µg of micronized chitosan, 1 µg of OVA, 3 µg of MPL, 3 µg of poly(I:C), and 3 µg of α-GalCer was obtained. Moreover, a test solution MCα3 was obtained which contained 3 µg of MPL, 3 µg of CpG and 3 µg of α-GalCer instead of 3 µg of MPL, 3 µg of poly(I:C), and 3 µg of α-GalCer. Moreover, a test solution PCα3 was obtained which contained 3 µg of poly(I:C), 3 µg of CpG and 3 µg of α-GalCer instead of 3 µg of MPL, 3 µg of poly(I:C), and 3 µg of α-GalCer.

For immunizing mice, 100 µl of the test solution MP3 describe in Example 6 was administered to each of the mice in the group 1, 100 µl of the test solution MC3 described in Example 6 was administered to each of the mice in the group 2, 100 µl of the test solution PC3 described in Example 6 was administered to each of the mice in the group 3, 100 µl of the test solution MPα3 was administered to each of the mice in the group 4, 100 µl of the test solution MCα3 was administered to each of the mice in the group 5, and 100 µl of the test solution PCα3 was administered to each of the mice in the group 6. Each of those administrations were intraperitoneally carried out twice at an interval of 2 weeks. Blood was collected from tail veins one week after the each immunization, and blood serum samples were thus obtained.

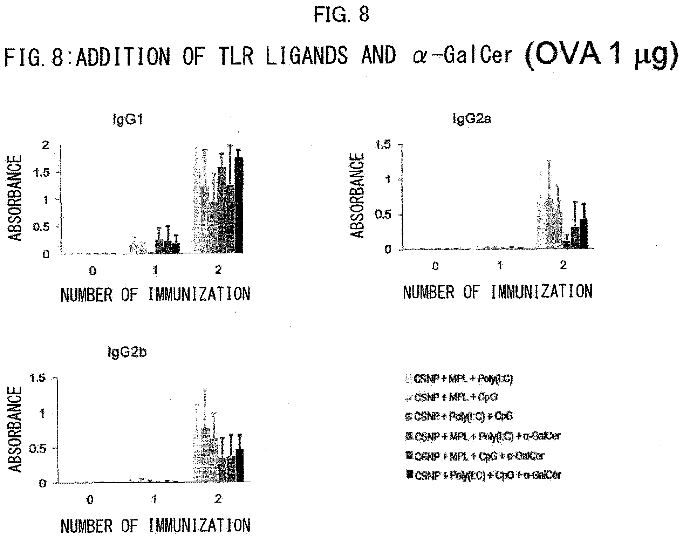

Except for the above points, an operation similar to that in Example 1 was carried out. The results are shown in Fig. 8.

### [Example 8]

An operation similar to that in Example 7 was carried out, except that serum samples were taken from tail veins 6, 12, and 24 hours after the second immunization and cytokine titers were measured instead of antibody titers.

0The cytokine titers were measured as follows. First, with use of an ELISA kit (Quantikine M 000Kit available from R&D Systems; IL-4, Mouse, ELISA Kit: M4000B; IL-2, Mouse, ELISA Kit: M2000; IFN-γ, Mouse, ELISA Kit: MIF00), each of the serum samples which were 5-fold diluted was added to each well in an amount of 100 µl/well, and was caused to react for 2 hours at a room temperature.

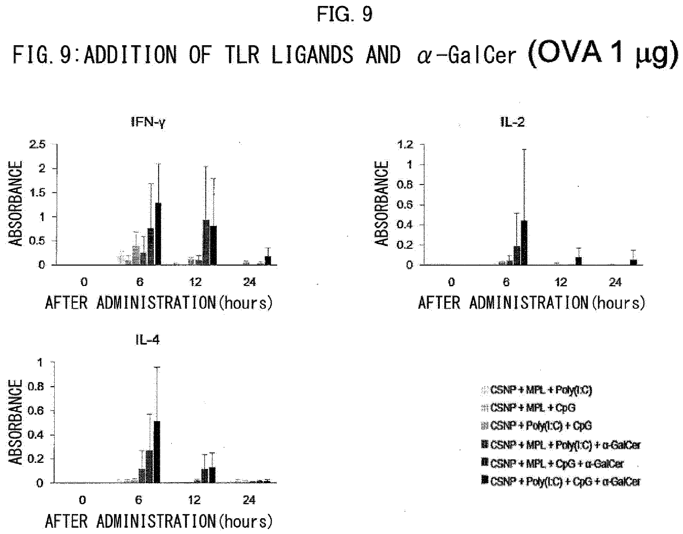

As a secondary antibody, an HRP-labeled anti-mouse IL-4 antibody (available from R&D Systems, Cat. No.: 892701), an HRP-labeled anti-mouse IL-2 antibody (available from R&D Systems, Cat. No.: 890328), or an HRP-labeled anti-mouse IFN-γ antibody (available from R&D Systems, Cat. No.: 892666) was used and caused to react for 2 hours at a room temperature. TMBZ (available from Sigma, Cat. No.: T3405) was used as a substrate and caused to react for 20 minutes at a room temperature, and then the reaction was terminated with 2N sulfuric acid. Absorbance (OD value) was measured with use of a plate reader (available from BIO-RAD: model 680, Cat No.: 168-1000) (dual: measured 450 nm, control 570 nm). The results are shown in Fig. 9.

### [Example 9]

The solution A obtained in Example 1 was stored at -20°C for one week. At the time of use, 1 µl of OVA as an antigen and any of TLR agonists were added to the solution A, and thus test solutions each of which was in an amount of 100 µl and contained 10 µg of micronized chitosan, 1 µg of OVA, and 3 µg of a TLR agonist were obtained. As the TLR agonists, 3 µl of MPL, 3 µg of poly(I:C), and 3 µg of CpG were used, respectively. Moreover, as a control, a solution in which all solutions were mixed at the time of use was obtained for each TLR agonist.

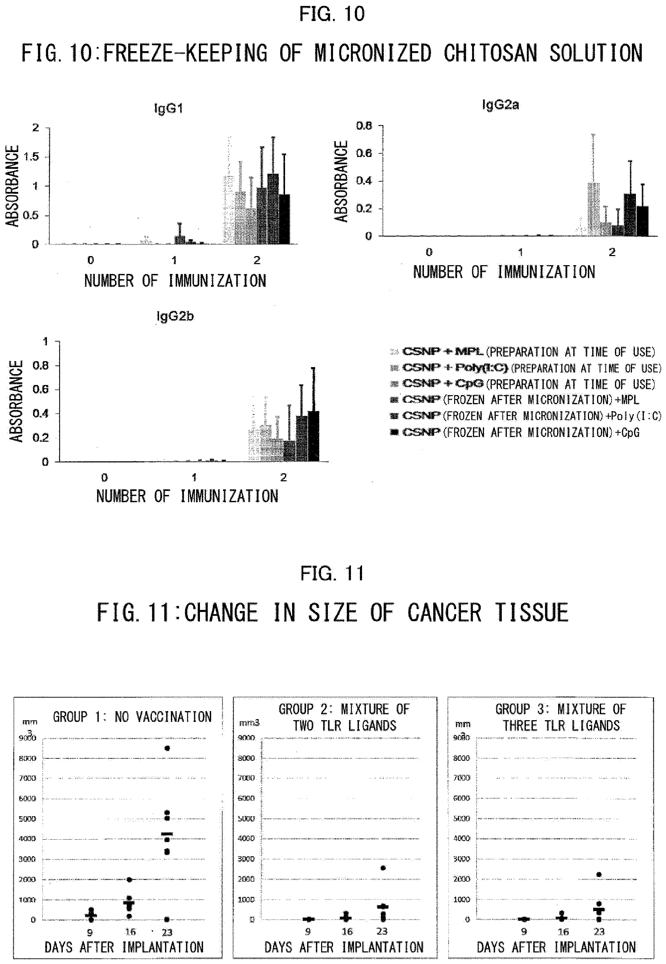

Antibody titers were measured by immunizing mice with use of a method similar to that of Example. The results are shown in Fig. 10.

### [Example 10]

Seven-week-old female C57BL/6NJcl mice (CLEA Japan, Inc.) were randomly selected into groups such that each group would include seven mice, after one week of habituation.
A cell suspension of EG7-OVA cells (as cancer cells; ATCC CRL-2113 (registered trademark)) in which OVA genes were introduced into EL4 lymphoma cells was mixed with an equal quantity of matrigel basement membrane matrix (available from Corning, Cat. No.: 354277) under ice cooling, and 5 × 10⁵ cells (liquid amount of 0.1 ml) were transplanted subcutaneously into a mouse.

Cancer vaccination was carried out according to the following prescription. The cancer vaccine was administered subcutaneously 7 days before cancer cell transplantation, and 2, 4 and 6 days after the transplantation.
Group 1: No vaccination
Group 2: 30 µg of micronized chitosan + 10 µg of CpG + 10 µg of poly(I:C) + 10 µg of α-GalCer + 30 µg of OVA
Group 3: 30 µg of micronized chitosan + 10 µg of CpG + 10 µg of poly(I:C) + 10 µg of MPL + 10 µg of α-GalCer + 30 µg of OVA

A size of a cancer tissue was measured 9, 16 and 23 days after the transplantation of the cancer cells. The size of the cancer tissue was measured by measuring a long diameter and a short diameter with a vernier caliper, and is expressed in a value of long diameter × short diameter²/2. The results are shown in Fig. 11.

The graphs in Fig. 1 show IgG1 production, IgG2a production, and IgG2b production in the order of upper left, upper right, and lower left, respectively. Fig. 1 shows that, when MPL was added, the productions of IgG2a and IgG2b became remarkable and the production of IgG1 was also enhanced at the small dose, i.e., 10 µg or 1 µg. Note that, in the drawings subsequent to Fig. 1, CS represents chitosan, NaOl represents sodium oleate, and OVA represents ovalbumin. In the drawings subsequent to Fig. 2, both CSNP and CS nanoparticles represent micronized chitosan.

The graphs of Fig. 2 show IgG1 production, IgG2a production, IgG2b production, and IgE production in the order of upper left, upper right, lower left, and lower right, respectively. Fig. 2 shows that, by increasing the dosage amount of OVA from 1 µg to 10 µg, the productions of IgG2a and IgG2b were clearly detected even when the micronized chitosan was used alone. This effect was further enhanced by the addition of MPL. In any of the conditions, the IgE production was low.

The graphs in each of Figs. 3 through 8 and 10 show IgG1 production, IgG2a production, and IgG2b production in the order of upper left, upper right, and lower left, respectively.

The graphs in Fig. 11 show the size of the cancer tissue. The left column shows the group 1, the center column shows the group 2, and the right column shows the group 3.

Fig. 3 shows that the productions of IgG1, IgG2a and IgG2b were greatly enhanced with the addition of the small dose, i.e., 3 µg to 30 µg of poly(I:C). This seems to be because the receptor TLR3 which receives poly(I:C) is present mainly in endosomes in the antigen presenting cells, and therefore the antigen and the adjuvant are efficiently taken into the antigen presenting cells in a state in which the antigen and the adjuvant are integrated.

Fig. 4 shows that the productions of IgG2a and IgG2b were greatly enhanced with the addition of the small dose, i.e., 1.5 µg to 15 µg of CpG. This seems to be because CpG was efficiently delivered to the receptor TLR9 which was a receptor for CpG and was present in endosomes in the antigen presenting cells.

Fig. 5 shows that the productions of IgG1, IgG2a, and IgG2b were enhanced with the addition of R848.

Fig. 6 shows that the combination of two substances among MPL, poly(I:C), and CpG further enhanced the productions of IgG1, IgG2a and IgG2b, as compared to cases where those substances were added alone.

Fig. 7 shows that, when α-GalCer was added, the productions of IgG1, IgG2a, and IgG2b were enhanced.

Fig. 9 shows that productions of IFN-γ, IL-2, and IL-4 were particularly strongly induced by the combination of CSNP + CpG + poly(I:C) + α-GalCer and the combination of CSNP + MPL + CpG + α-GalCer. A peak production of the cytokines was at 6 hours after the second immunization.

From Figs. 1 through 9, it can be seen that the immunostimulator which contains the micronized chitosan, the TLR ligand and α-GalCer stimulates both humoral immunity (lgG1) and cell-mediated immunity (IgG2a and IgG2b).

As seen in Fig. 10, with any of the TLR ligands, the results of adding the TLR ligand at the time of use after the solution, which had been prepared and in which the micronized chitosan was suspended, was kept frozen were similar to the result of adding all the components at the time of use.

From Fig. 11, it can be seen that the administration of vaccine inhibits enlargement of cancer tissue.

### [Example 11]

Subsequent to Example 10, an influence of a combination of micronized chitosan (containing sodium oleate (nacalai tesque, 25702-82)) and the TLR ligand exerted on a tumor was further analyzed. Differences from Example 10 are summarized below.

### (a) Administration substance

Test article 1 (group 2): Micronized chitosan + OVA + CpG
Test article 2 (group 3): Micronized chitosan + OVA + poly(I:C)
Test article 3 (group 4): Micronized chitosan + OVA + MPLA
Test article 4 (group 5): Micronized chitosan + OVA + R848
Test article 5 (group 6): Micronized chitosan + OVA + CpG + poly(I:C) + α-GalCer
   Control article 1 (group 1): Not administered
   Control article 2 (group 9): Micronized chitosan + OVA
   Control article 3 (group 10): Adjuvant 1 + OVA
   Control article 4 (group 11): Adjuvant 2 + OVA

### (b) Composition of administration substance

In the above (a), "OVA" represents Ovalbumin EndoFit (InvivoGen), "CpG" represents ODN1688 Class B, Cat. No.: tlrl-1668-1 (InvivoGen), and "R848" represents CL097, Cat. No.: tlrl-C97 (InvivoGen).

The test article 5 was substantially identical with the group 2 described in Example 10, except that the amount of OVA was reduced from 30 µg to 10 µg, the amounts of CpG and poly(I:C) were reduced from 10 µg to 3 µg, and the amount of α-GalCer was reduced from 10 µg to 1 µg. The test articles 1 through 4 were substantially identical with the test article 5, except that only one PAMP (i.e., CpG, poly(I:C), MPLA or R848) was used (in the same amount, i.e., 3 µg) and α-GalCer was not used.

The control article 1 corresponded to test articles 1 through 4 containing no micronized chitosan, no OVA, and no PAMP. The adjuvant 1 was a conventional adjuvant (aluminum hydroxide gel; Imject (registered trademark) Alum Adjuvant (Thermo Fisher Scientific)) at 2 mg (i.e., the upper limit of preparable concentration), which was used instead of 30 µg of micronized chitosan. The adjuvant 2 was a conventional adjuvant (aluminum hydroxide gel; LG-6000 (LSL)) which was used in an amount of 30 µg (same amount as FL-80) instead of 30 µg of micronized chitosan.

### (c) Test schedule

The table below summarizes types of processes animals are to undergo and dates on the basis of the administration day (0d) of tumor cells. "VA" in the table is an abbreviation for "vaccine administration" and represents the administration of the above administration substance to the animals.

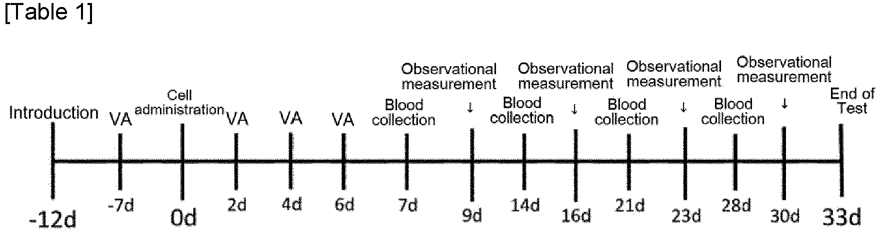

### (d) Site of administration in animal

According to the descriptions in Example 10, the cancer cells were transplanted subcutaneously (dorsal left side) in the animals, and the administration substances were administered intradermally (dorsal right side) in the animals. Each of the groups 1 through 11 included seven animals on the day 0d in the above table.

### (e) Results

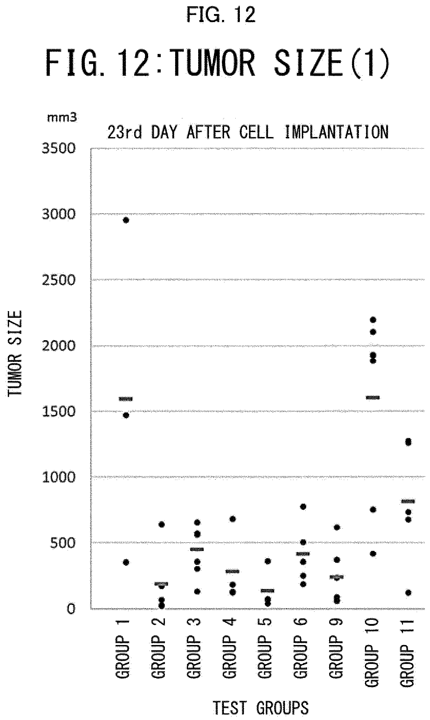

The results of the tests carried out according to (a) through (d) above are shown in Fig. 12. As shown in Fig. 12, the test article 5 (group 6) shows, as compared to the control article 1, that PAMP (TLR ligand) in the amount smaller than that of Example 10 is enough to achieve the tumor-shrinking effect. The test articles 1 through 4 (groups 2 through 5) show, as compared to the test article 5, that a single TLR ligand is needed to achieve the tumor-shrinking effect. The test articles 1 through 4 show that α-GalCer is not necessarily needed to achieve the tumor-shrinking effect.

The test articles 1 through 4 show that CpG and R848 are most excellent in tumor-shrinking effect as a single TLR, followed by MPLA and poly(I:C) in this order. Comparisons of the test articles 1 through 5 to the control article 2 show that an appropriate selection of the type of the TLR ligand added to the micronized chitosan contributes to improvement of the tumor-shrinking effect. Note that control articles 1 through 4 show that the micronized chitosan is more effective for tumor shrinkage, as compared to the adjuvants 1 and 2.

### [Example 12]

Effectiveness (in view of tumor shrinkage and elevation of blood antibody titer) of a combination of micronized chitosan containing a chitosan derivative (mannose-modified FL-80) and a TLR ligand was further analyzed. Differences from Example 11 are described below. IgG in blood was measured according to the method described in Example 1, except that serum samples were 8000-fold diluted and an HRP-labeled anti-mouse IgG antibody (Nichirei Corporation, Code: 424131) was used as a secondary antibody.

### (a) Administration substance

Test article 1 (group 2): Micronized chitosan + MPLA
Test article 2 (group 3): Micronized chitosan + CpG
Test article 3 (group 4): Micronized chitosan + poly(I:C)
   Control article 1 (group 1): Not administered
   Control article 2 (group 5): Micronized chitosan + α-GalCer
   Control article 3 (group 6): Micronized chitosan

### (b) Test schedule

The table below summarizes types of processes animals are to undergo and dates on the basis of the administration day (0d) of tumor cells. Blood was collected after completion of observational measurements (47d) and blood antibody titers were measured.

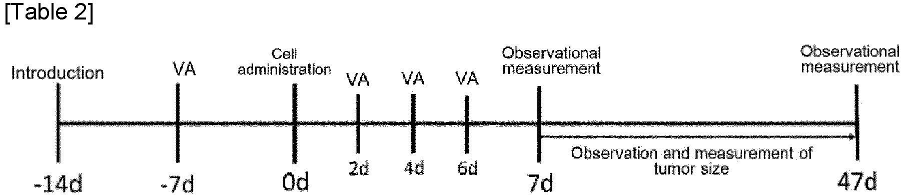

### (c) Results

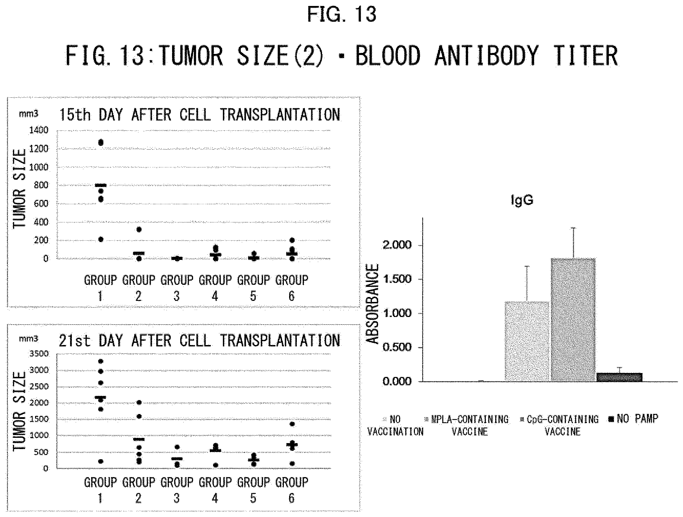

Effectiveness of the chitosan derivative was analyzed and the result thereof is shown in Fig. 13. As shown in the upper left and the lower left of Fig. 13, the chitosan derivative exhibited the tumor-shrinking effect at degrees varying according to the type of TLR ligand (particularly in the test articles 2 and 3), as with chitosan FL-80. As indicated in the right of Fig. 13, the chitosan derivative exhibited elevation of blood antibody titer, as with chitosan FL-80.

The present invention can be used as an immunostimulator.

## Claims

1. An immunostimulator comprising:
- particulate chitosan; and
- a toll-like receptor (TLR) ligand.

2. The immunostimulator as set forth in claim 1, further comprising an anionic surfactant.

3. The immunostimulator as set forth in claim 2, wherein the anionic surfactant is at least one selected from the group consisting of phospholipids, C10-C22 fatty acids, and salts of the C10-C22 fatty acids.

4. The immunostimulator as set forth in claim 3, wherein:
- the phospholipids are lecithin or lysolecithin; and
- the C10-C22 fatty acids and the salts of the C10-C22 fatty acids are sodium oleate or sodium laurate.

5. The immunostimulator as set forth in any one of claims 1 through 4, wherein the particulate chitosan has a weight-average molecular weight of 5k or more and 1000k or less, and has a particle diameter of 500 nm or less.

6. The immunostimulator as set forth in any one of claims 1 through 5, wherein the TLR is at least one selected from the group consisting of TLR3, TLR4, TLR7, TLR8, and TLR9 of human.

7. The immunostimulator as set forth in any one of claims 1 through 6, further comprising an antigen.

8. The immunostimulator as set forth in any one of claims 1 through 7, further comprising α-GalCer.

9. A kit for producing an immunostimulator, said kit comprising:
- chitosan which is contained in a container and retains an anionic surfactant; and
- an instruction manual for said kit,
- the chitosan being particulate chitosan, and
- the instruction manual indicating that the immunostimulator is produced by causing the particulate chitosan to retain a TLR ligand.

10. A vaccine comprising an immunostimulator recited in any one of claims 1 through 8.

11. The vaccine as set forth in claim 10, wherein said vaccine is a cancer vaccine.

12. A method for producing an immunostimulator, said method comprising a step of causing particulate chitosan to retain a TLR ligand.
